# EUROPEAN PATENT APPLICATION

(11) **EP 1 118 330 A1**
(43) Date of publication of application: **25.07.2001**
(21) Application number: 00400132.7
(22) Date of filing: 19.01.2000
(51) Int. Cl.: A61K 38/21, A61P 35/00

(54) **Interferon-alpha use in the treatment of Ewing's family of tumors**

(71) Applicant: INSTITUT NATIONAL DE LA SANTE ET DE LA RECHERCHE MEDICALE (INSERM), 75654 Paris Cédex 13 (FR); Institut Curie, 75005 Paris (FR)
(72) Inventor: Sanceau, Josiane, 94800 Villejuif (FR); Wietzerbin, Jeanne, 91160 Antony (FR)
(74) Representative: Le Guen, Gérard

(57) **Abstract**

The invention relates to the use of interferon-alpha for the treatment of Ewing's family of tumors in mammals. A method of treating Ewing's family of tumors in a mammal comprising administering to the mammal in need of such treatment a therapeutically effective amount of interferon-alpha is disclosed. The present invention further relates to pharmaceutical compositions suitable for administration to a mammal for the treatment of Ewing's family of tumors comprising interferon-alpha in a therapeutically effective amount, together with a pharmaceutically acceptable carrier.

## Description

The invention relates to the use of interferon-alpha for the treatment of Ewing's family of tumors in mammals.

The family of Ewing's tumors represents the second most common type of bone tumors observed in children after osteosarcoma. The family includes Ewing's sarcoma (EWS or Ewing's tumor of bone), extraosseus Ewing's (EOE), primitive neuroectodermal tumors (PNET or peripheral neuroepithelioma) and Askin's tumors (PNET of the chest wall). These tumors are rare diseases in which malignant cells are found in the bone and soft tissues, and recent protocols use the same treatment for this family of tumors. EWS is mostly observed in adolescent and young adults, and the most common sites for the primary lesion are the pelvic bones, femur, humerous and ribs. Incidence of Ewing's sarcoma is estimated to be about 60% of the Ewing's family of tumors.

Studies using immunohistochemical markers, cytogenetics, molecular genetics, and tissue culture indicate that these tumors are all derived from the same primordial stem cell. Cytogenetic studies of the Ewing's family of tumors have identified a consistent alteration of the EWS locus on chromosome 22 band q12 that may involve other chromosomes, including 11 or 21. Characteristically, the amino terminus of the EWS gene is juxtaposed with the carboxy terminus of another chromosome. In the majority of cases (90%), this chromosomal translocation directs the fusion of the 5'end of the EWS gene, encoding a protein capable of participation in RNA metabolism, with the 3' end of the Fli-1 gene, a member of the Ets transcription factor family located on chromosome 11 band q24 (Delattre *et al*., *Nature 359*:162-165 (1992)). Several fusion genes can be generated according to the cleavage sites (between exons 7 and 11 of EWS and exons 3 to 9 from Fli-1).

The more frequent fusion, EWS-Fli-1 type I, fuses EWS exon 7 with Fli-1 exon 6 and represents 50% of cases. The type II fusion (25% of cases) ligates EWS exon 7 with Fli-1 exon 5. In addition to these two principal fusion types, about ten other combinations have been described.

In certain Ewing's tumor cases, EWS is not fused to Fli-1 but to another member of the Ets family, *e.g.* FEV. Other Ets family members which may combine with the EWS gene are ERG (located on chromosome 21), ETV (located on chromosome 7), and E₁AF (located on chromosome 17), which result in the following translocations: t(21;22), t(7;22), and t(17;22) respectively.

These genetic alterations reach a constant biochemical consequence: Ewing cells always express a chimeric protein bearing the N-terminal region of EWS fused to a DNA binding domain of the Ets protein family. This constancy suggests that this fusion exerts a central role in Ewing's tumor development.

In Ewing's sarcoma, the role of the EWS/Fli-1 fusion protein has been experimentally demonstrated. This protein can transform mice fibroblasts, and these cells are able to generate tumors in nude mice.

From *in vitro* experiments, it was shown that the EWS/Fli-1 protein was able to activate certain gene transcription more efficiently than Fli-1. This fact suggests that the EWS/Fli-1 protein fusion exerts its oncogenic actions through the abnormal activation of certain cellular genes.

It was also shown that the PU-1 protein, also from the Ets family, is implicated in the control of interferon and cytokine regulated gene expression (Perez *et al*., *Mol. Cell Biol. 14*:5023-5031 (1994)).

Important prognostic factors for the Ewing's family of tumors include the site and volume of the primary tumor and whether the disease is metastatic. Size of the tumor is also thought to be an important variable. With current treatment, studies suggest that 50%-70% of patients without metastatic disease have a long-term disease-free survival, compared to only 20%-30% for patients who present with metastatic disease. As such, there remains an acute need in the art for an effective method of treating the Ewing's family of tumors.

Accordingly, the present invention provides an effective method of treating the Ewing's family of tumors in a mammal. Other features and advantages of the present invention will be set forth in the detailed description of preferred embodiments that follows, and in part will be apparent from the description or may be learned by practice of the invention. These advantages of the invention will be realized and attained by the compositions and methods particularly pointed out in the written description and claims hereof.

It has been discovered that interferon-alpha (IFN-α) has an antiproliferative action on Ewing's sarcoma. Therefore, one embodiment of the present invention is directed to a method of treating Ewing's family of tumors in a mammal comprising administering to a mammal in need of such treatment a therapeutically effective amount of IFN-α. A second embodiment of the present invention is directed to a composition suitable for administration to a mammal for the treatment of Ewing's family of tumors comprising IFN-α in a therapeutically effective amount, together with a pharmaceutically acceptable carrier.

It is to be understood that both the foregoing general description and the following detailed description are exemplary and explanatory only and are intended to provide further explanation of the invention as claimed.

Unless defined otherwise, all technical and scientific terms used herein are intended to have the same meaning as is commonly understood by one of ordinary skill in the relevant art.

As used herein, the term "Ewing's family of tumors" means a type of tumor found in bone and soft tissues characterized by small round cells and associated with an alteration of the EWS locus on chromosome 22. The family of tumors includes Ewing's sarcoma (Ewing's tumor of bone or ETB), extraosseus Ewing's (EOE), peripheral primitive neuroectodermal tumors (PNET or peripheral neuroepithelioma) and Askin's tumors (PNET of the chest wall).

The individual cells of Ewing's sarcoma and EOE contain round to oval nuclei with fine dispersed chromatin without nucleoli. Occasionally, cells with smaller, more hyperchromatic (and probably degenerative) nuclei are present giving a "light cell-dark cell" pattern. The cytoplasm varies in amount, but in the classic case it is clear and contains glycogen, which can be highlighted with a periodic acid-Schiff (PAS) stain. The tumor cells are tightly packed and grow in a diffuse pattern without evidence of structural organization.

The histologic appearance of the PNET differs somewhat from Ewing's sarcoma and EOE. These tumors are typically composed of round to ovoid hyperchromatic cells with minimal cytoplasm. The tumor cells are typically arranged in nests and trabeculae with variable rosette formation. The rosettes may have a central lumen, but are often ill-defined, composed of tumor cells arranged around an empty space. The classic lobular growth pattern is best appreciated at low-power, and differs from the typical diffuse growth seen in EOE. Occasionally, groups of cytologically uniform, round cells with dispersed chromatin resembling those in EOE are seen interspersed in an otherwise typical PNET. This overlap of features lends confidence to the concept that these tumors are indeed the same tumor with a spectrum of differentiation.

As used herein, the term "mammal" indicates any mammalian species, and includes, but is not limited to, rabbits, mice, dogs, cats, primates and humans, preferably humans. As used herein, the term "treatment" refers to any process, action, application, therapy, or the like, wherein a mammal, including a human being, is subject to medical aid with the object of improving the mammal's condition, directly or indirectly. In the context of tumor growth or tumor cell growth, "treatment" includes, but is not limited to, inhibition and prevention.

As used herein, the term "inhibition" can be assessed by the delayed appearance of primary or secondary tumors, slowed development of primary or secondary tumors, decreased occurrence of primary or secondary tumors, slowed or decreased severity of secondary effects of disease, arrested tumor growth and regression of tumors, among others. In the extreme, complete inhibition is referred to herein as prevention.

As used herein, the term "prevention" means no tumor or tumor cell growth if none had occurred, and no further tumor or tumor cell growth if there had already been growth.

As used herein, the term "therapeutic treatment" indicates treating a subject after a disease is contracted, and includes prophylactic therapy.

As used herein, the term "effective amount" signifies an amount effective to perform the function specified. A "therapeutically effective amount," in reference to the treatment of a tumor, refers to an amount sufficient to bring about one or more or the following results: reduce the size of the tumor, inhibit the metastasis of the tumor, inhibit the growth of the tumor, prevent the growth of the tumor, relieve discomfort due to the tumor, or prolong the life of a patient inflicted with the tumor.

As used herein, the term "interferon" means the family of highly homologous cytokine proteins and glycoproteins which are known to have various biological activities, such as antiviral, antiproliferative and immunomodulatory activity at least in the species of animal from which such substances are derived.

At present, the interferons are categorized into five different types: alpha IFN (leukocyte IFN), beta IFN (fibroblast IFN), gamma IFN (immune IFN), omega IFN and tau IFN (trophoblastic factor). For a review of the details and homology relationships of the known IFN proteins, *see, e.g*., Viscomi, *Biotherapy 10*:59-86 (1997).

As used herein, the terms "interferon-alpha" and "IFN-α" include all proteins, polypeptides and peptides which are natural or recombinant IFN-α's or derivatives thereof, and which are characterized by the biological activity of those IFN-α's against malignant, non-malignant or viral diseases. These include IFN-α-like compounds from a variety of sources such as natural IFN-α's (human and non-human), recombinant IFN-α's and synthetic or semi-synthetic IFN-α's.

IFN-α is itself a mixture of proteins with very extensive homology encoded by a family of several different structural genes. These subtypes have an identical functional spectrum but their specific activities are different. More than 20 different subtypes of IFN-α having distinct amino acid sequences have been identified by isolating and sequencing DNA encoding these peptides, including IFN-α2a, 2b and 2c, for example. Most of the species have a signal peptide sequence of 23 amino acid residues and a mature amino acid sequence of 166 amino acid residues (Goeddel, D. V. *et al., Nature 290*:20-26 (1981); Weissmann, C. and Weber, *H., Prog. Nuc. Acid Res. Mol. Biol. 33*:251-300 (1986); Zoon, K. C., *Interferon 9*:1-12 (1987)). The amino acid sequence and recombinant preparation of IFN-α-2a and IFN-α-2b are described, for example, in European Patent Publication No. 0 043 980, and in European Patent Publication No. 0 321 134, respectively.

As used herein, the term "recombinant interferon-alpha" refers to IFN-α produced by recombinant DNA techniques, *i.e.,* produced from cells transformed by an exogenous DNA construct encoding the desired IFN-α polypeptide.

As used herein, the term "International Unit" means the internationally established potency unit of measurement of IFN as defined by the International Conference for Unification of Formulae and as recognized by those of skill in the art.

As used herein, the term "pharmaceutically acceptable carrier" refers to a diluent, adjuvant, excipient, or vehicle with which the therapeutic is administered. It includes any and all solvents, dispersion media, aqueous solutions, coatings, antibacterial and antifungal agents, isotonic and absorption delaying agents, and the like. The use of such media and agents for pharmaceutical active substances is well known in the art. Except insofar as any conventional media or agent is incompatible with the active ingredient, use thereof in the pharmaceutical compositions is contemplated. Supplementary active ingredients can also be incorporated into the compositions of the invention. A composition is said to be "pharmacologically acceptable" if its administration can be tolerated by a recipient patient.

As used herein, "simultaneous or sequential" is meant that IFN-α is co-administered with another cytokine or chemotherapeutic agent or together with radiotherapy or surgery or where IFN-α administration is preceded or followed by non-IFN-α treatment. Where "sequential" therapy is occurring, the time difference between IFN-α administration and non-IFN-α treatment can be minutes, hours, days, weeks or months depending on the tumor or sarcoma being treated, the mammal being treated and the effectiveness of the overall treatment.

The term "substantially simultaneously" means "at about the same time" with the only limitation being that both substances must be present together at the tumor site.

In accordance with the present invention, it has been discovered that IFN-α can significantly inhibit and/or prevent tumor growth in Ewing's sarcoma. As such, in one aspect of the invention there is provided a method of treating Ewing's family of tumors in a mammal comprising administering to the mammal in need of such treatment a therapeutically effective amount of IFN-α.

IFN-α was among the first of the cytokines to be produced by recombinant DNA technology and its properties, chemical nature and methods of preparation have been extensively studied and documented. In general, the IFN-α of the present invention can be prepared or obtained from a variety of sources including natural sources, biochemical synthesis and purification and recombinant DNA techniques, such as those using synthetic genes expressed in *E. coli (see* U.S. Patent No. 5,710,027). *See also* Pestka, "*Interferon alpha"* in Human Cytokines, Blackwell Scientific Publications 1-16 (1992).

In a preferred embodiment, the IFN-α is recombinant IFN-α. For example, recombinant IFN-α1a, 2a (commercialized as Laroferon® or Roferon A® by Hoffman La Roche or as Inter-A-2® by Bio Sidus) or 2b (commercialized under the name of INTRON A®, Viraferon® by Schering Plough or as Bioferon® by Bio Sidus) can be used. More preferably, recombinant IFN-α2a is utilized.

An extracted natural IFN-α can be used in the present invention as well. Natural human IFN-α has been purified from several cell sources including leukocytes isolated from whole blood, neonatal fibroblasts, lymphoblastoid and various leukemic cell lines. IFN-α can also be a mammalian extract such as ruminant or bovine IFN-α. As such, the IFN-α employed can be "homologous" to the mammal to be treated meaning that it has the same origin as the species of mammal to be treated (*e.g.* human IFN-α for treatment of a human or murine IFN-α for treatment of a mouse) or can be "heterologous" to the mammal to be treated meaning that the species origin of IFN-α and the species of the mammal are different (*e.g.* human IFN-α for treatment of a mouse or murine IFN-α for treatment of a human).

The IFN-α of the inventive method also includes, for example, known sequences and those variants whose genes are characterized by a high degree of homology with the known sequence and which code for biologically active IFN-α and compounds having substantially the same biologically activity as known forms of IFN-α. The IFN-α can also contain single or multiple amino acid insertions, deletions and/or additions to the naturally occurring sequence and may be fragmented to a part carrying the active site of IFN-α.

In general, any INF-α molecule is encompassed by the present invention and is included in the expression "IFN-α" provided all such molecules have the effect of preventing or reducing the size of Ewing's family of tumors *in vitro,* in a laboratory test animal *in vivo* or in a mammal to be treated.

The present invention is exemplified by the effect of IFN-α against Ewing's sarcoma tumors in mice. It is to be understood, however, that the present invention extends to the *in vivo* effects of IFN-α in all mammals such as humans, and to all of Ewing's family of tumors. In a preferred embodiment, the mammal is human. Exemplary non-human mammals include livestock animals such as cows, pigs, sheep, horses, goats, companion animals such as cats and dogs, and laboratory test animals such as mice, rabbits, guinea pigs or hamsters.

According to the present invention, the IFN-α can be administered by any suitable route such as oral or parenteral, *i.e.,* any route other than the alimentary canal. In a preferred embodiment, the parenteral administration is by intravenous (within or into a vein), intramuscular (within a muscle), subcutaneous (introduced beneath the skin) or percutaneous (effected through the skin) means. A systematic injection is preferred wherein IFN-α enters the blood stream, but an injection in the tumor itself can also be advantageously used.

Administration can be as a single dose or repeated doses one or more times after a certain period. When administering IFN-α by injection, the administration may be by continuous injections, or by single or multiple boluses. The administration modes and posology can be determined by those skilled in the art according to criteria generally considered for a therapeutical treatment adapted to a patient. As such, dosage of the administered agent will vary depending upon such factors as the patient's age, weight, height, sex, general medical condition, previous medical history, *etc.*

The effective amount of IFN-α will depend on the mammal and the condition to be treated. In general, it is desirable to provide the recipient with a dosage which is in the range of about 1 x 10⁶ to about 90 x 10⁶ International Units (IU), although a lower or higher dosage may be administered. Preferably, a dose of in the range of about 1 x 10⁶ to about 45 x 10⁶ IU can be administered. Skilled practitioners will adjust the timing and dosage to fit the clinical symptoms of the patients. Such knowledge has been accumulated over decades and is reported in the medical literature. The IFN-α composition may be administered alone or formulated with pharmaceutically acceptable carriers, in either single or multiple doses. As such, in another embodiment of the invention the IFN-α is administered as part of a pharmaceutical composition comprising a pharmaceutically acceptable carrier.

Suitable pharmaceutical carriers include, but are not limited to, inert solid diluents or fillers, sterile aqueous solutions, and various nontoxic organic solvents. Pharmaceutically acceptable carriers should be inert. Further, the carrier should not react with or otherwise reduce the effectiveness of the active ingredients, and more particularly, should not significantly diminish the effectiveness or stability of the IFN-α. Pharmaceutically acceptable carriers include water, ethanol, polyethylene glycol, mineral oil, petrolatum, propylene glycol, lanolin, and similar agents.

The pharmaceutical forms suitable for injectable use include sterile aqueous solutions (where water soluble) or dispersions and sterile powders for the extemporaneous preparation of sterile injectable solutions or dispersion. In all cases the form should be sterile and should be fluid to the extent that easy syringability exists. It should also be stable under the conditions of manufacture and storage and be preserved against the contaminating action of microorganisms such as bacteria and fungi.

In another embodiment of the invention, the IFN-α composition is administered alone or in combination with another pharmaceutical agent, *i.e*., "combined therapy." IFN-α can be administered with other cytokines and/or chemotherapeutic agents and/or radiotherapeutic protocol and/or with surgery. Such cytokines include, but are not limited to, interleukin-1, TNF-α and/or interferon-gamma. Representative chemotherapeutic agents are well known in the art and include, but are not limited to, Doxorubicin, Actimomycin, Cyclophosphamide (VAdriaC), Etoposide, Ifosfamide and Vincristine.

Amounts of other cytokines will be similar to the effective amounts of IFN-α. The effective amounts of chemotherapeutic agents will vary depending on the agent and are known in the art. The subject compositions may be administered either in conjunction with the second treatment modalities, or separately, *e.g.* at different times or in different syringes or tablets.

Surgery may be used in certain cases to try to remove the cancer and some of the tissue around it. Surgery may also be used to remove any tumor that is left after chemotherapy or radiation therapy. In a preferred embodiment, the IFN-α composition is administered in combination with surgical therapy.

Another aspect of the present invention further comprises the substantially simultaneous or sequential administration of one or more other cytokines, derivatives thereof and/or one or more chemotherapeutic agents and/or the simultaneous or sequential treatment by radiotherapy. Accordingly, IFN-α can be co-administered with another cytokine or chemotherapeutic agent or together with radiotherapy or where IFN-α administration is preceded or followed by non-IFN-α treatment. Where "sequential" therapy is occurring, the time difference between IFN-α administration and non-IFN-α treatment may be minutes, hours, days, weeks or months depending on the tumor or sarcoma being treated, the mammal being treated and the effectiveness of the overall treatment.

The present invention further relates to the use of IFN-α or active fragment, mutant or derivative thereof alone or together with one or more other cytokines and/or chemotherapeutic agents in the manufacture of a medicament for the treatment of patients carrying tumors or sarcomas from the Ewing's family of tumors.

The present invention also relates to a process for preparing the above-mentioned pharmaceutical composition characterized by mixing, according to known methods, active ingredients with acceptable excipients which are pharmaceutically acceptable.

In all of the above cases, the present invention also extends to the use of derivatives of IFN-α and derivatives of other cytokines and/or chemotherapeutic agents. By "derivative" is meant recombinant, chemical or other synthetic forms of IFN-α or other cytokine or chemotherapeutic agent and/or any alteration such as addition, substitution and/or deletion to the amino acid sequence component of the molecule, provided the derivative possesses the ability to prevent and/or inhibit Ewing's family of tumors.

The present invention further extends to a pharmaceutical composition comprising IFN-α and/or its derivatives alone or in combination with one or more other cytokines and/or their derivatives and/or chemotherapeutic agents and one or more pharmaceutically acceptable carriers and/or diluents.

All patents and publications cited in this disclosure are indicative of the level of skill of those skilled in the art to which this invention pertains and are all herein incorporated by reference in their entirety.

Having now generally described the invention, the same will be more readily understood through reference to the following Examples which are provided by way of illustration, and are not intended to be limiting of the present invention, unless specified.

### LEGENDS OF FIGURES

Figure 1 represents the effect of IFN-α on Ewing's tumor development as generated by subcutaneous injection of EW-7 cells to mice. Relative tumor volumes (RTV) are measured on a time dependent manner.

Figure 2 displays a growth rate plot of the Ewing's tumors grown in nude mice after IFN-α injection. The average value of relative tumor volumes (RTV) of 12 mice is represented.

Figure 3 represents the effect of IFN-α on Ewing's tumors grown in nude mice. Tumor volumes were determined twice a week for each group, and the volume median was determined.

### EXAMPLES

### Example 1 : Malignant Cell Injection

Solid tumors were generated by subcutaneous injection of 20 x 10⁶ EW7 cells (shoulder-blade tumor derived cell line) from primary tumor before chemotherapy (EW exon 7/ Fli exon 6) in 6 week-old nude mice.

After ten days, two groups of five mice each were constituted, a control mice group and a mice group receiving IFN-α (r-hu-α2a from Hoffman La Roche).

Injections were performed in the tumor itself with 5 x 10⁵ IFN units per mouse, 3 times weekly. Treatment was continued for a 5-week period during which tumor volumes were determined every 3 days.

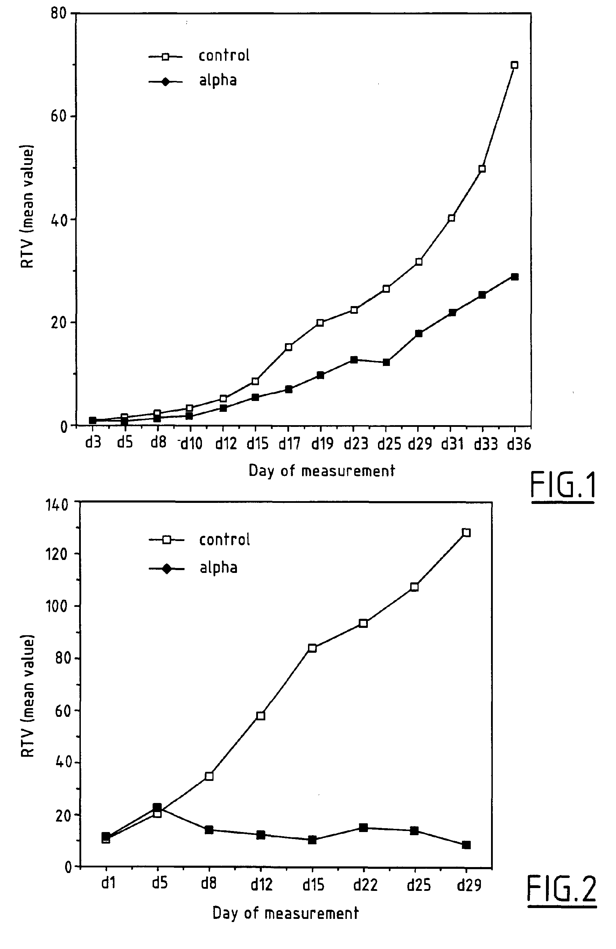

Relative tumor volumes (RTV) demonstrate slower tumor growth after IFN-α injection. Figure 1 constructed from RTV average dates shows a very sharp decrease of turnover growth rates after IFN-α injection.

These results from established tumors in the experimental growth phase permit the confirmation of *in vitro* results with cell lines.

### Example 2 : Solid Tumor Graft

A second experiment was performed *in vivo* in 8-week old nude mice. A tumor sample 5 mm in diameter from a solid tumor developed in a nude mouse after 20 x 10⁶ EW7 cells injections, conserved by *in vivo* transfer, was grafted in nude mice.

Three days after grafting, two groups of 12 mice each were constituted, a control group receiving phosphate buffer solution (PBS) injections and a group receiving IFN-α injections. 1 x 10⁶ units of IFN-α was injected in tumor sites in a 5 days-a-week manner (Monday through Friday) for 1 month.

The tumor growth curves were determined after measuring grafted tumor diameters in each mouse twice a week (Figure 2).

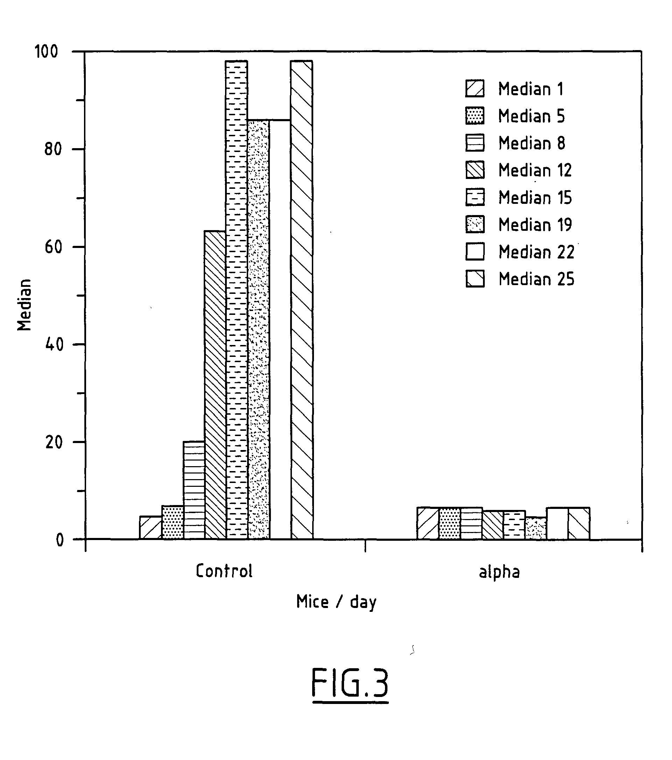

Figure 2 shows the plots obtained from average RTV for each 12 mice group. An almost linear progression of tumor development in control mice was observed, while tumor proliferation was practically null in grafts receiving IFN-α. This result was confirmed by median calculation for each group (Figure 3).

It is worth noting that one month after the interruption of IFN injection, 40% of the mice which had received IFN-α did not develop tumors.

In view of the foregoing description taken with the Examples, those skilled in the art will be able to practice the invention in various enablements and embodiments without departing from the spirit and scope of the invention as defined in the appended claims.

## Claims

1. Use of interferon-alpha for the preparation of a medicament for the treatment of Ewing's family of tumors.

2. Use according to claim 1, wherein the tumor is Ewing's sarcoma.

3. Use according to any of claims 1 or 2, wherein said interferon-alpha is interferon-alpha-2a.

4. Use according to any of claims 1 to 3, wherein said interferon-alpha is recombinant interferon-alpha.

5. Use according to any of claims 1 to 4, wherein said interferon-alpha is meant to be administered orally or parenterally.

6. Use according to claim 5, wherein said parenteral administration is by intramuscular, intravenous, subcutaneous or percutaneous administration.

7. Use according to claim 5, wherein said interferon-alpha is meant to be administered by systematic or direct tumor injection.

8. Use according to any of claims 1 to 7, wherein said interferon-alpha is meant to be administered in an effective amount from about 1 to about 90 million International Units, preferably from about 1 to about 45 million International Units.

9. Use according to any of claims 1 to 8, wherein said interferon-alpha is meant to be administered simultaneously or sequentially, together with one or more agents used in the treatment of Ewing's family of tumors.
